(19) Europäisches Patentamt
European Patent Office
Office européen des brevets

(11) Veröffentlichungsnummer: **0 136 702**
**B1**

(12) # EUROPÄISCHE PATENTSCHRIFT

(45) Veröffentlichungstag der Patentschrift: **09.05.90**

(21) Anmeldenummer: **84111800.3**

(22) Anmeldetag: **03.10.84**

(51) Int. Cl.$^5$: **C 07 C 251/04,**
C 07 D 309/06,
C 07 D 213/52,
C 07 D 335/02,
C 07 D 307/14,
C 07 D 261/18,
C 07 D 493/04,
C 07 D 405/12,
C 07 D 407/12,
C 07 D 409/12, A 01 N 35/10

(54) **Cyclohexenol-Derivate, Verfahren zu ihrer Herstellung und ihre Verwendung zur Bekämpfung unerwünschten Pflanzenwuchses.**

(30) Priorität: **05.10.83 DE 3336140**
**25.11.83 DE 3342630**

(43) Veröffentlichungstag der Anmeldung:
**10.04.85 Patentblatt 85/15**

(45) Bekanntmachung des Hinweises auf die Patenterteilung:
**09.05.90 Patentblatt 90/19**

(84) Benannte Vertragsstaaten:
**AT BE CH DE FR GB IT LI NL SE**

(56) Entgegenhaltungen:
**EP-A-0 070 370**
**EP-A-0 080 301**
**EP-A-0 082 694**
**EP-A-0 104 876**
**GB-A-2 125 042**
**CHEMICAL ABSTRACTS, Band 86, 1977, Seite 397, Nr. 16357h, Columbus, Ohio, US; & JP - A - 76 76 245 (NIPPON SODA CO., LTD.) 01.07.1976**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Jahn, Dieter, Dr.**
**Burgunder Weg 8**
**D-6803 Edingen-Neckarhausen (DE)**
Erfinder: **Becker, Rainer, Dr.**
**Im Haseneck 22**
**D-6702 Bad Duerkheim (DE)**
Erfinder: **Keil, Michael, Dr.**
**Fontanestrasse 4**
**D-6713 Freinsheim (DE)**
Erfinder: **Schirmer, Ulrich, Dr.**
**Berghalde 79**
**D-6900 Heidelberg (DE)**
Erfinder: **Richarz, Winfried, Dr.**
**Koenigsberger Strasse 5**
**D-6081 Stockstadt (DE)**
Erfinder: **Wuerzer, Bruno, Dr. Dipl.-Landwirt**
**Ruedigerstrasse 13**
**D-6701 Otterstadt (DE)**
Erfinder: **Meyer, Norbert, Dr.**
**Dossenheimer Weg 22**
**D-6802 Ladenburg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 136 702 B1

**Beschreibung**

Die Erfindung betrifft Cyclohexenol-Derivate, Verfahren zu ihrer Herstellung sowie Herbizide, die diese Verbindungen als Wirkstoffe enthalten.

Es ist bekannt, Cyclohexenol-Derivate zur Bekämpfung von unerwünschten Gräsern in breitblättrigen Kulturen anzuwenden (DE—A 24 61 027).

Weitere Angaben zu Cyclohexenol- und/oder Cyclohexandionverbindungen, die als Herbizide verwendet werden können, finden sich in den EP—A—70 370, 80 301, 82 694, 104 876, der GB—A—2 125 042, der US—A—4 422 864 und in C.A. 1977, Bd. 86, S. 397.

Es wurde gefunden, daß Cyclohexenol-Derivate der Formel

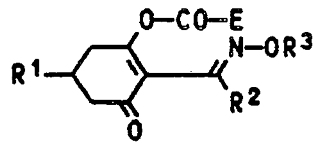

(I),

in der

$R^1$ einen 6-gliedrigen Heterocyclus, enthaltend 1 Ringglied aus der Gruppe O und S, oder einen Rest der allgemeinen Struktur B—Y—C—, wobei B $C_1$—$C_4$-Alkyl, Y Sauerstoff oder Schwefel und C eine Alkylenkette mit maximal 4 Kohlenstoffatomen bedeuten,

$R^2$ Alkyl mit 1 bis 4 Kohlenstoffatomen,

$R^3$ Alkyl mit 1 bis 3 Kohlenstoffatomen, Alkenyl mit 3 oder 4 Kohlenstoffatomen, Halogenalkenyl mit 3 oder 4 Kohlenstoffatomen und 1 bis 3 Halogensubstituenten oder Propargyl und

E einen gegebenenfalls durch maximal 3 Substituenten aus der Gruppe Halogen und $C_1$—$C_4$-Alkyl im Phenoxyteil substituierten Phenoxyalkylenrest mit einer gegebenenfalls verzweigten Alkylenkette mit maximal 5 Kohlenstoffatomen bedeuten, herbizid wirksam sind. Sie sind, je nach zu bekämpfendem Pflanzenspektrum und je nach Verträglichkeit für Kulturpflanzen, entweder selektive Herbizide oder dienen generell zur Bekämpfung und Eindämmung unerwünschter Vegetation, eingeschlossen die Unterdrückung des Wachstums von vegetativen und generativen Pflanzenteilen bei Kulturpflanzen, wo solche unbrauchbar, hinderlich oder gar nachteilig sind.

$R^1$ in Formel I bedeutet beispielsweise einen Heterocyclus, wie 5,6-Dihydro-2H-pyranyl-, Tetrahydropyranyl-, 5,6-Dihydro-2H-thiopyranyl-, Tetrahydrothiopyranyl- oder 1,3-Dioxepanylreste, z.B. 5,6-Dihydro-2H-pyran-3-yl, Tetrahydropyran-3-yl, 5,6-Dihydro-2H-thiopyran-3-yl, Tetrahydrothiopyran-3-yl.

$R^1$ kann weiterhin einen Rest der allgemeinen Struktur B—Y—C—, wobei B für eine $C_1$—$C_4$-Alkyl-Rest, Y für Sauerstoff oder Schwefel und C für eine Alkylenkette mit maximal 4 Kohlenstoffatomen stehen, bedeuten. Beispiele hierfür sind 2-Ethylthio-n-propyl, 2-Methoxy-n-propyl, 1-Methyl-2-methoxyethyl.

$R^2$ in Formel I steht für unverzweigte und verzweigte Alkylreste mit 1 bis 4 Kohlenstoffatomen, d.h. für Methyl, Ethyl, n-Propyl, i-Propyl, n-Butyl, sec.-Butyl, i-Butyl, tert.-Butyl.

Reste für $R^3$ in Formel I sind Propargyl, Alkyl mit 1 bis 3 Kohlenstoffatomen, Alkenyl mit 3 oder 4 C-Atomen oder Halogenalkenyl mit 3 oder 4 C-Atomen, das bis zu drei Halogensubstituenten enthalten kann, beispielsweise Methyl, Ethyl, n-Propyl, i-Propyl, n-Butyl, sec.-Butyl, i-Butyl, tert.-Butyl, Allyl, 1-Chlorprop-1-en-3-yl, 2-Chlorprop-1-en-3-yl, 1,3-Dichlorprop-1-en-3-yl, 1,1,2-Trichlorprop-1-en-3-yl.

E kann einen gegebenenfalls durch maximal 3 Substituenten aus der Gruppe Halogen oder $C_1$—$C_4$-Alkyl im Phenoxy-Teil substituierten Phenoxyalkylenrest bedeuten, wie Phenoxymethyl, Chlorphenoxymethyl, Dichlorphenoxymethyl, Chlor-methyl-phenoxymethyl, Trichlorphenoxymethyl, Methylphenoxyethyl, Dichlorphenoxyethyl, Chlor-methylphenoxyethyl, Trichlorphenoxyethyl, Chlorphenoxypropyl, Dichlorphenoxypropyl.

Cyclohexenol-Derivate der Formel I können durch Umsetzung von entsprechenden Verbindungen der Formel

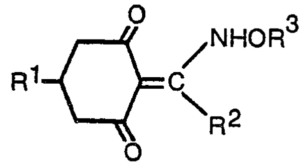

(II),

mit Verbindungen der Formel ZCOE, wobei E die obengenannten Bedeutungen hat und Z einen Halogen- oder Tosylrest bedeutet, erhalten werden. Die Umsetzung wird in Gegenwart einer Base bei Temperaturen von −5°C bis zum Siedepunkt des Gemisches in einem inerten Lösungsmittel durchgeführt. Gegebenenfalls kann die Base in wäßriger Lösung eingesetzt werden. Je nach Mischbarkeit wird dann die Reaktion in homogener Phase oder im Zweiphasensystem durchgeführt. Im letzteren Fall können zur Beschleunigung der Reaktion Phasentransfer-Katalysatoren, wie Ammonium- und Phosponiumsalze, eingesetzt werden.

Außerdem können zur Beschleunigung der Reaktion Azolverbindungen, wie Imidazol, Pyrazol, Pyridin sowie seine Derivate, z. B. 4-Piperidinopyridin oder 4-Dimethylaminopyridin, verwendet werden.

Geeignete Lösungsmittel sind Dimethylsulfoxid, Dimethylformamid, Benzol, Toluol, gegebenenfalls chlorierte Kohlenwasserstoffe, wie Chloroform, Dichlormethan, Dichlorethan, Hexan, Cyclohexan, Ketone, wie Aceton, Butanon, Ester, wie Essigsäureethylester, Ether, wie Diethylether, Dioxan, Tetrahydrofuran.

Geeignete Basen sind beispielsweise Carbonate, Hydrogencarbonate, Acetate, Alkoholate, Hydroxide oder Oxide von Alkali- oder Erdalkalimetallen, insebesondere von Natrium, Kalium, Magnesium, Calcium. Außerdem können auch organische Basen, wie Pyridin oder tertiäre Amine, Verwendung finden.

Als Ausgangsprodukte zur Synthese der erfindungsgemäßen Verbindungen können auch Salze von Verbindungen der Formel II dienen. Die Umsetzung erfolgt dann in oben angegebener Weise ohne Zusatz von Basen.

Zu Verbindungen der Formel II gelangt man nach bekannten Verfahren:

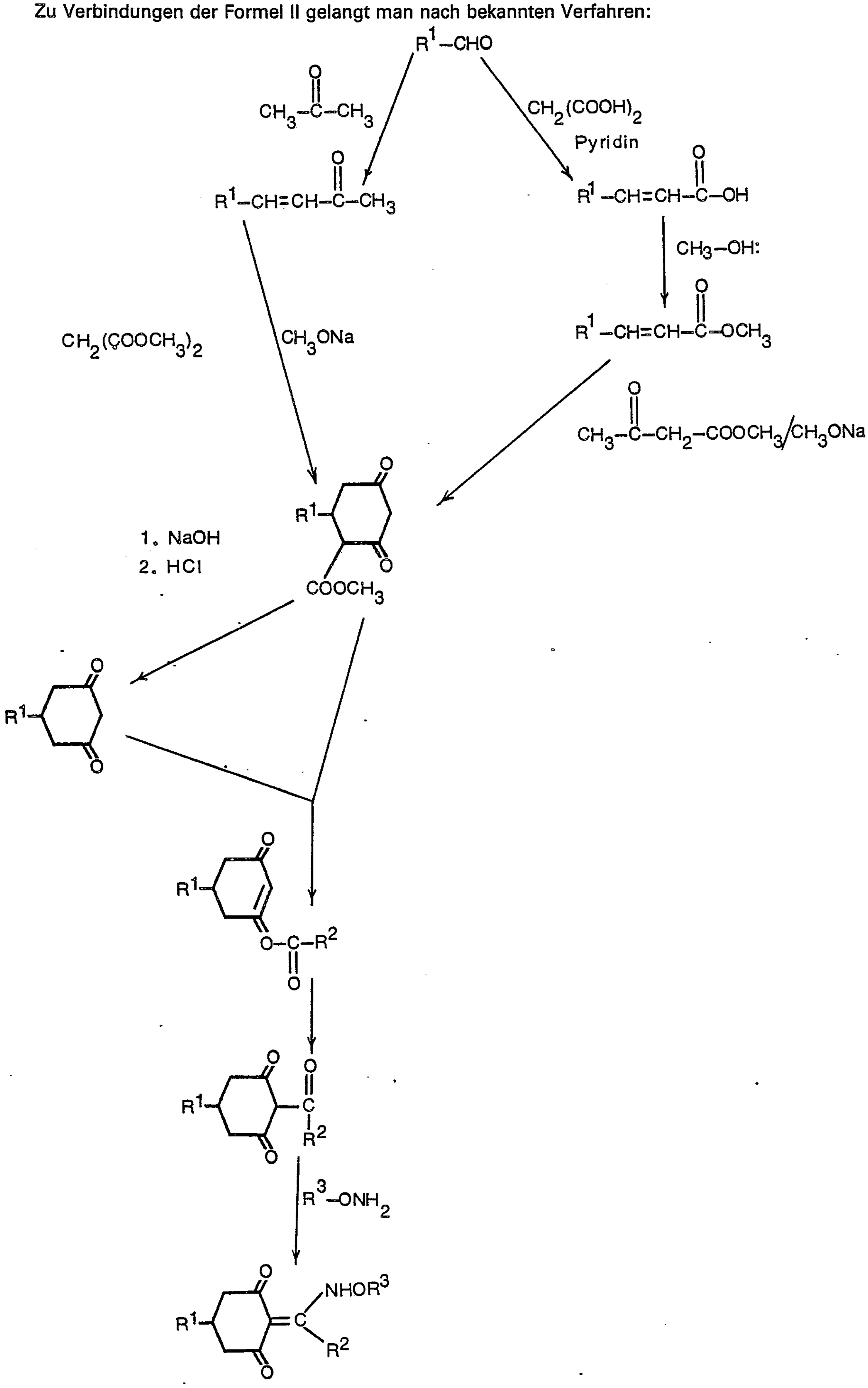

Die folgenden Beispiele erläutern die Herstellung der neuen Cyclohexenol-Derivate der Formel I.

In den Beispielen verhalten sich Gewichtsteile zu Volumenteilen wie Kilogramm zu Liter.

Die $^1$H-NMR-Daten sind in δ-Werten (ppm) angegeben und auf Tetramethylsilan als internem Standard bezogen. Als Lösungsmittel diente $CDCl_3$. Abkürzungen für die Signalstrukturen: s = Singulett, d = Dublett, t = Triplett, q = Quartett, m = Multiplett, stärkstes Signal.

Beispiel 1

3,1 Gewichtsteile 2-Ethoxyaminobutyliden-5-(tetrahydropyran-3-yl)-cyclohexan-1,3-dion, 1,2-Gewichtsteile Triethylamin und 0,2 Gewichtsteile 4-Dimethylaminopyridin werden in 50 Volumenteilen Dichlormethan auf 0°C gekühlt. Zu dieser Lösung werden bei Temperaturen zwischen 0°C und 5°C 1,4 Gewichtsteile Benzoylchlorid getropft. Anschließend läßt man die Mischung auf Raumtemperatur kommen, rührt noch eine Stunde, wäscht mit 10prozentiger Salzsäure, zweimal mit 5prozentiger Natronlauge und Wasser, trocknet über Natriumnsulfat und destilliert das Lösungsmittel ab. Man erhält 1-Benzoyloxy-2-(N-ethoxybutyrimidoyl)-5-(tetrahydropyran-3-yl)-cyclohex-1-en-3-on als Öl. (Wirkstoff Nr. 1) $^1$H-NMR-Spektrum 0,92 (t), 3,95 (t), 7,65 (m).

| Wirkstoff Nr. | $R^1$ | $R^2$ | $R^3$ | -CO-E | $^1H$-NMR-Daten |
|---|---|---|---|---|---|
| 2 | Tetrahydropyran-3-yl | n-Propyl | Ethyl | 2-(4-Chlor-2-methyl-phenoxy)-propionyl | 1,25 (t), 4,85 (q), 7,15 (m) |
| 3 | Tetrahydropyran-3-yl | n-Propyl | Ethyl | 4-(2,4-Dichlor-phenoxy)-butyryl | 1,60 (m), 2,17 (m), 7,18 (m) |
| 4 | 2-Ethylthio-n-propyl | n-Propyl | Ethyl | 2-(4-Chlor-2-methyl-phenoxy)-propionyl | 0,87 (t), 2,55 (m), 7,1 (m) |
| 5 | 2-Ethylthio-n-propyl | n-Propyl | Ethyl | 4-(2,4-Dichlor-phenoxy)-butyryl | 0,85 (t), 4,05 (m), 7,16 (m) |
| 6 | 2-Ethylthio-n-propyl | n-Propyl | Ethyl | 2,4-Dichlorphenoxy-acetyl | |
| 7 | Tetrahydrothiopyran-3-yl | n-Propyl | Ethyl | 2-(4-Chlor-2-methyl-phenoxy)-propionyl | |
| 8 | Tetrahydrothiopyran-3-yl | n-Propyl | Ethyl | 4-(2,4-Dichlorphenoxy)-butyryl | |
| 9 | Tetrahydropyran-3-yl | n-Propyl | Ethyl | 2-(2,4,5-Trichlor-phenoxy)-propionyl | 4,85 (m), 7,01 (s), 7,52 (s) |
| 10 | 2-Ethylthio-n-propyl | n-Propyl | Ethyl | 2-(2,4,5-Trichlor-phenoxy)-propionyl | 4,10 (q), 7,00 (s), 7,52 (s) |

Die Cyclohexenol-Derivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittleren Naphthalinen oder Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Poloxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinnusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoff an festen Trägerstoffen hergestellt werden. Feste Trägerstoffe sind Mineralerden wie Silicagel, Kieselsäuren, Kieselgele, Silikate, Talkum, Kaolin, Attaclay, Kalksteine, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Die Applikation kann im Vorauflaufverfahren oder im Nachlauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,025 bis 3 kg/ha, vorzugsweise 0,1 bis 1,5 kg/ha.

Die Wirkung der Cyclohexenol-Derivate der Formel I auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 1,5% Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmaßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erste bis zu einer Wuchshöhe von 3 bis 15 cm an und behandelte sie danach. Die Sojapflanzen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung betragen 0,125, 0,25, 0,5 und 3,0 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Alopecurus myosuroides (Ackerfuchsschwanz), Avena fatua (Flughafer), Avena sativa (Hafer), Beta vulgaris (Zuckerrübe), Brassica napus (Raps), Bromus spp. (Trespearten), Cassia spp., Digitaria sanguinalis (Blutfingerhirse), Echinochloa crus-galli (Hühnerhirse), Eleusine indica, Glycine max (Sojabohnen), Gossypium hirsutum (Baumwolle), Helianthus annuus (Sonnenblume), Hordeum vulgare (Gerste), Ipomoea spp. (Prunkwindearten), Lolium multiflorum (Ital. Raygras), Setaria italica (Kolbenhirse), Sinapis alba (Weißer Senf), Sorghum bicolor (Mohrenhirse; Kulturhirse), Sorghum halepense (Sudangras; Wilde Mohrenhirse), Triticum aestivum (Weizen), Zea mays (Mais).

Die Verbindungen Nr. 2, 4, 5 und 9 bekämpfen bei Vorauflaufanwendung sowohl die grasartigen Beispielspflanzen als auch den breitblättrigen Senf.

In Anbetracht des erfaßbaren Wirkunbgsspektrums zur Unkrautbekämpfung, der Verträglichkeit für Kulturpflanzen oder der unerwünschten Beeinflussung des Wachstums derselben sowie angesichts der Vielfalt der Applikationsmethoden können die erfindungsgemäßen Verbindungen in einer großen Zahl von Kulturpflanzen eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var, silvestris | Rübsen |
| Camellia sinensis | Teestrauch |
| Carthamus tinctorius | Saflor — Färberdistel |
| Caryg illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |

| Botanischer Name | Deutscher Name |
|---|---|
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glyoine max | Sojabohne |
| Gossypium hirsutum (Gossypium arboreum Gossypium hebaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |

| Botanischer Name | Deutscher Name |
|---|---|
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum bicolor (s. vulgare) | Mohrenhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais (Unterblatt oder post-directed) |

Zur Verbreiterung des Wirkungsspektrums und zur Erzielung synergistischer Effekte können die neuen substituierten Cyclohexenol-derivate sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren und andere in Betracht.

Außerdem kann es von Nutzen sein, die Cyclohexenol-Derivate der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernähungs- und Spurenelementmängeln eingesetzt werden. Es können auch nicht-phytotoxische Öle und Ölkonzentrate zugesetzt werden.

**Patentansprüche**

1. Cyclohexenol-Derivate der Formel

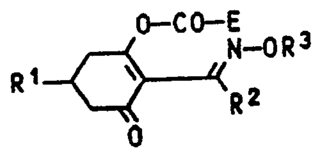

(I),

in der

$R^1$ einen 6-gliedrigen Heterocyclus, enthaltend 1 Ringglied aus der Gruppe O und S oder einen Rest der allgemeinen Struktur B—Y—C—, wobei B $C_1$—$C_4$-Alkyl, Y Sauerstoff oder Schwefel und C eine Alkylenkette mit maximal 4 Kohlenstoffatomen bedeuten,

$R^2$ Alkyl mit 1 bis 4 Kohlenstoffatomen,

$R^3$ Alkyl mit 1 bis 3 Kohlenstoffatomen, Alkenyl mit 3 oder 4 Kohlenstoffatomen, Halogenalkenyl mit 3 oder 4 Kohlenstoffatomen und 1 bis 3 Halogensubstituenten oder Propargyl und

E einen gegebenenfalls durch maximal 3 Substituenten aus der Gruppe Halogen und $C_1$—$C_4$-Alkyl im Phenoxyteil substituierten Phenoxyalkylenrest mit einer gegebenenfalls verzweigten Alkylenkette mit maximal 5 Kohlenstoffatomen bedeutet.

2. Cyclohexenol-Derivate der Formel I gemäß Anspruch 1, dadurch gekennzeichnet, daß $R^1$ einen Tetrahydropyranyl-, Tetrahydrothiopyranyl-, 1-Oxo-tetrahydrothiopyranyl- oder 1,1-Dioxo-tetrahydrothiopyranylrest bedeutet.

3. Cyclohexenol-Derivate der Formel I gemäß Anspruch 1, dadurch gekennzeichnet, daß $R^1$ einen Tetrahydrothiopyran-3-ylrest bedeutet.

4. 1-Benzoyloxy-2-(N-ethoxybutyrimidoyl)-5-(tetrahydrothiopyran-3-yl)-cyclohex-1-en-3-on und 1-Benzoyloxy-2-(N-ethoxybutyrimidoyl)-5-(tetrahydropyran-3-yl)-cyclohex-1-en-3-on.

5. Verfahren zur Herstellung eines Cyclohexenol-Derivates der Formel I gemäß Anspruch 1, dadurch gekennzeichnet, daß man eine entsprechende Verbindung der Formel

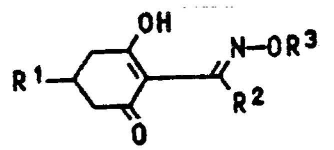

(II),

mit einer entsprechenden Verbindung ZCOE in Gegenwart einer Base umsetzt, wobei E die in Anspruch 1 genannten Bedeutungen hat und Z einen Halogen- oder Tosylrest bedeutet.

6. Herbizid, enthaltend inerte Zusatzstoffe und ein Cyclohexenol-Derivat der Formel I gemäß einem der Ansprüche 1 bis 4.

**Revendications**

1. Dérivés de cyclohexénol de formule

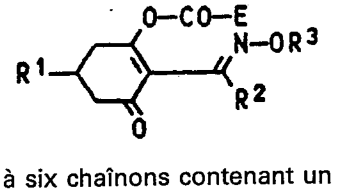

(I),

dans laquelle

$R^1$ représente un hétérocycle à six chaînons contenant un chaînon du groupe 0 et S ou un reste de structure générale B—Y—C, B représentant alkyle en $C_1$—$C_4$, Y, oxygène ou soufre et C, une chaîne alkylène à 4 atomes de carbone au maximum,

$R^2$, alkyle à 1 à 4 atomes de carbone,

$R^3$, alkyle à 1 à 3 atomes de carbone, alcényle à 3 ou 4 atomes de carbone, halogénoalcényle à 3 ou 4 atomes de carbone et 1 à 3 substituants halogène, ou propargyle, et

E, un reste phénoxyalkylène à chaîne alkylène éventuellement ramifiée, ayant au maximum 5 atomes de carbone, éventuellement substitué dans la partie phénoxy par 3 substituants au maximum, choisis dans le groupe halogène et alkyle en $C_1$—$C_4$.

2. Dérivés de cyclohexénol de formule I, selon la revendication 1, caractérisés par le fait que $R^1$ représente un reste tétrahydropyranyle, tétrahydrothiopyranyle, 1-oxo-tétrahydrothiopyranyle ou 1,1-dioxotétrahydrothiopyranyle.

3. Dérivés de cyclohexénol de formule I, selon la revendication 1, caractérisés par le fait que $R^1$ représente un reste tétrahydrothiopyran-3-yle.

4. 1-benzoyloxy-2-(N-éthoxybutyrimidoyl)-5-(tétrahydrothiopyran-3-yl)-cyclohex-1-én-3-one et 1-benzoyloxy-2-(N-éthoxybutyrimidoyl)-5-(tétrahydropyran-3-yl)-cyclohex-1-én-3-one.

5. Procédé de préparation d'un dérivé de cyclohexénol de formule I, selon la revendication 1, caractérisé par le fait que l'on fait réagir, en présence d'une base, un composé approprié de formule

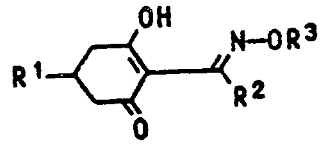 (II),

avec un composé approprié ZCOE, E ayant les seignifications données dans la revendication 1 et Z représentant un reste halogène ou tosyle.

6. Herbicide, contenant des additifs inertes et un dérivé de cyclohexénol de formule I, selon l'une des revendications 1 à 4.

**Claims**

1. A cyclohexenol derivative of the formula

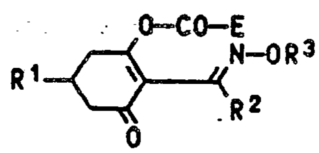 (I),

where $R^1$ is a 6-membered heterocyclic structure containing one ring member from the group consisting of O and S, or a radical of the general structure B—Y—C—, where B is $C_1$—$C_4$-alkyl, Y is oxygen or sulfur and C is an alkylene chain of not more than 4 carbon atoms, $R^2$ is alkyl of 1 to 4 carbon atoms, $R^3$ is alkyl of 1 to 3 carbon atoms, alkenyl of 3 or 4 carbon atoms, haloalkenyl of 3 or 4 carbon atoms and 1 to 3 halogen substituents or propargyl, and E is phenoxyalkylene which is unsubstituted or substituted in the phenoxy moiety by not more than 3 substituents from the group consisting of halogen and $C_1$—$C_4$-alkyl and which possesses a straight-chain or branched alkylene chain of not more than 5 carbon atoms.

2. A cyclohexenol derivative of the formula I as claimed in claim 1, where $R^1$ is tetrahydropyranyl, tetrahydrothiopyranyl, 1-oxotetrahydrothiopyranyl or 1,1-dioxotetrahydrothiopyranyl.

3. A cyclohexenol derivative of the formula I as claimed in claim 1, where $R^1$ is tetrahydrothiopyran-3-yl.

4. 1-Benzoyloxy-2-(N-ethoxybutyrimidoyl)-5-(tetrahydrothiopyran-3-yl)-cyclohex-1-en-3-one and 1-benzoyloxy-2-(N-ethoxybutyrimidoyl)-5-(tetrahydropyran-3-yl)-cyclohex-1-en-3-one.

5. A process for the preparation of a cyclohexenol derivative of the formula I as claimed in claim 1, wherein a corresponding compound of the formula

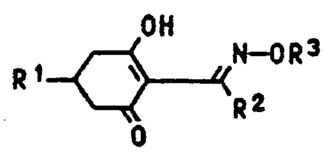 (II),

is reacted with a corresponding compound ZCOE, where E has the meanings given in claim 1 and Z is halogen or tosyl, in the presence of a base.

6. A herbicide containing inert additives and a cyclohexenol derivative of the formula I as claimed in any of claims 1 to 4.